Europäisches Patentamt

**European Patent Office**

**Office européen des brevets**

(11) Publication number: **0 253 554**
A2

# EUROPEAN PATENT APPLICATION

(21) Application number: **87305979.4**

(22) Date of filing: **07.07.87**

(51) Int. Cl.4 **A61K 9/22**

(30) Priority: **15.07.86 US 885975**

(43) Date of publication of application:
**20.01.88 Bulletin 88/03**

(84) Designated Contracting States:
**AT BE CH DE ES FR GB GR IT LI LU NL SE**

(71) Applicant: **PFIZER INC.**
**235 East 42nd Street**
**New York, N.Y. 10017(US)**

(72) Inventor: **Curatolo, William John**
**18, Patrick Place**
**Niantic State of Connecticut(US)**

(74) Representative: **Moore, James William, Dr.**
**Pfizer Limited Ramsgate Road**
**Sandwich Kent CT13 9NJ(GB)**

(54) **Controlled release drug-containing fibers.**

(57) Orally administerable drug-containing fibers for controlling drug release in the gastrointestinal tract of a mammal.

EP 0 253 554 A2

## CONTROLLED RELEASE DRUG-CONTAINING FIBERS

This invention relates to drug-containing fibers for the controlled release of said drug in the gastrointestinal tract of a mammal. More particularly it relates to orally administered drug-containing fibers which, because of their shape and dimensions, are retained in the proximal region of the gastrointestinal tract where they release said drug in a controlled manner to achieve a beneficial effect.

Means for achieving retention of drugs in the gastrointestinal tract and for controlled release of said drugs therein has been a long sought objective. Upon per os administration of a drug or drug preparation most, if not all of it, usually passes from the stomach to the small intestine in a relatively short time, generally on the order of one to two hours. This behavior necessitates frquent per os administration of a drug, the beneficial effect of which is to be exhibited in the stomach or the wall thereof. For some drugs, efficient absorption occurs only in the upper gastrointestinal tract, i.e., the stomach and or the small intestine. Slow release formulations of such drugs may only be effective for a short time period, generally 4-5 hours, because the formulation passes into the colon, where drug absorption may be inefficient or nonexistent. In such cases, retention of a slow release drug preparation in the upper gastrointestinal tract would be advantageous.

Various methods have been described in the literature in efforts to achieve retention and controlled release of drugs in the gastrointestinal tract.

U.S. Patent 3,976,764, issued August 24, 1976, describes hollow globular shells having an undercoating of a cellulose acetate-phthalate copolymer and an outer coating of ethyl and hydroxypropyl celluloses in combination with a pharmaceutically active ingredient. Said coated globular shells are reported to float in the gastric juices when taken internally to provide prolonged release of the active ingredient.

The effect of density upon the transit of pellets through the gastrointestinal tract is, at the present time, uncertain. Bechgaard et al., J. Pharm. Pharmacol. 30, 690-692 (1978), working with ileostomy subjects, reported that density rather than diameter was the major factor in achieving retention of a drug pellet in the small intestine. In a more recent study with ileostomy subjects, Bechgaard et al., J. Pharm. Pharmacol. 37, 718-721 (1985) reported that transit time of pellets was not affected by particle density. On the other hand, Meyer et al., Gastroenterology 89 , 805-13 (1985) reported that pellets with a density greater than one or less than one exhibited slower gastrointestinal transit time than pellets with a density of one.

EP Application 0168862 published January 22, 1986 describes biodegradable hollow fibers containing an active substance in their cavities for controlled release of said substance when implanted subcutaneously in mammals.

Davis et al ., Int. J. Pharm. 21, 331-340 (1984) teach that gastrointestinal transit of a pharmaceutical dosage form depends upon several factors such as size, shape and nature of the system; i.e., whether single unit or multiparticulate; and upon physiological factors, especially upon the presence or absence of food in the stomach. The stomach is known to empty different materials at different rates and to break down digestible materials to about 2mm or less before they pass through the pylorus into the duodenum. Davis et al. (loc . cit.) note that pharmaceutical dosage forms greater than 2 mm in size would be expected to remain in the stomach throughout the postprandial period if taken with or shortly before a meal and to pass from the stomach only when it was empty of food. Meals of high calorific value and certain foodstuffs, especially fats, appear to have an inhibitor effect on gastric emptying [Davis et al., Int. J. Pharma. 21, 167-177, (1984)].

Retention of indigestible materials in an empty stomach is further complicated by the ability of the gastrointestinal tract to undergo powerful contractions called the interdigestive myoelectric complex (IMC) or, more simply, "housekeeper wave". This phenomenon tends to sweep indigestible materials from an empty stomach past the pylorous into the duodenum and through the remainder of the small intestine.

Thus, retention of drugs or drug formulations in the proximal region of the gasstrointestinal tract in order for said drug or formulation to achieve its beneficial effect poses a difficult problem. Although Davis et al. (loc. cit.) found that an osmotic device, Osmet, available from Alza Corporation, Palo Alto, Ca., was retained in the stomach for approximately 10 hours when taken following a heavy breakfast, it was eliminated quite rapidly from the stomach when taken following a light breakfast. The need for means for achieving retention of drugs or drug formulations in the stomach for controlled (sustained, predictable and reproducible) release of the drugs over a prolonged time regardless of whether the stomach is full, empty or anywhere in between is highly desirable.

Mitra, Polymer Preprints, ACS Div. Polymer Chemistry, 24 (1), 51-52 (1983), describes an oral sustained release drug delivery system comprising a multilayered polymeric composite film. The composite is generally cut into long narrow strips 2.1 x 14 cm², optionally pleated before being packed into gelatin capsules. The polymeric composite film is a laminate comprising a carrier film containing drug in a matrix and a barrier film on one or both surfaces of the carrier film. The barrier film serves to control the rate of release of the drug and also provides buoyancy to the delivery system by virtue of air bubbles between it and the carrier film.

Films of the dimensions disclosed by Mitra will be recognized by those experienced in the art to be medically undesirable, in view of their potential to cause blockage of the gastrointestinal tract either at the pyloric value or, more likely, at the ileocaecal junction. Furthermore, gastric retention studies disclosed by Mitra demonstrate that the films disclosed are retained for only 6.5 hours in fed dogs. The fibers of the present invention are retained for much longer time periods in fed dogs (see Examples 5 and 6 below). However, the long-standing problem is development of a dosage form which will be retained in the stomach in the unfed state. The fibers of the present invention are clearly shown to be gastrically retained in the unfed state (see Examples 5, 6, 7, 8 below). The Mitra paper discloses no information related to the gastric retention of films in the unfed state.

U.S. Patent 4,165,998, issued August 28, 1979, discloses a method of manufacturing solid pharmaceutical unit dosage forms. The pharmaceutical is loaded on an edible web and the web fabricated into a solid geometric form of predetermined dimensions. While a variety of geometric forms are disclosed, fibers, such as are the subject of the present invention, are not mentioned. The preferred embodiment of the web dosage form is one which releases drug more quickly than a standard tablet or capsule. No disclosure relative to gastric retention or delayed gastrointestinal transit is made.

U.S. Patent 4,268,497, issued May 19, 1981, describes resilient and erodable ethylene-vinyl acetate sheets having a veterinary medicine dispersed therein and which are enclosed in an apertured flexible envelope. Said sheets are constrained in a rolled-up configuration prior to administration to a ruminant animal.

U.S. Patent 4,601,893, issued July 22, 1986, is directed to active agent-containing laminate devices perforated by one or more macroholes for controlled release of said agent to an ambient environment. The devices comprise sheets (having dimensions from 7-15 cm in length, 4-10 cm in width and 2-4 mm thickness for cattle and from 5-10 cm in length, 3-8 cm in width and 1-3 mm thickness for use in sheep) which are rolled and constrained into cylinders of from 0.8 cm to 3.0 cm in diameter.

U.S. Patent 4,455,146, issued June 19, 1984, discloses novel plasters comprising a thermoplastic elastomer, an oil or higher fatty acid, a tack-providing resin and a medicinal ingredient which is said to afford easy release from the body after application thereto.

European Patent Application 200,224, published November 5, 1986, discloses pharmaceutical compositions comprising a carrier and at least one drug delivery system consisting of a polymeric tube having a membrane outer sheath and a hollow drug-containing core. While tubes of up to 15 cm are disclosed as preferred, shorter lengths, e.g., less than 2 cm and preferably in the range of about 0.5 to 6 mm, are more preferred. Outside diameters of the tubes can range from 0.5, or less, to 5 mm. The ratio of tube length to tube diameter is in the range of about 1-30. The tubes are formulated in the form of tablets, capsules, suppositories, suspensions or sutures for administration to mammals. However, no disclosure or recognition of the use of drug-containing fibers as devices for retention in the gastrointestinal tract for controlled release of drugs are presented therein.

This invention concerns drug-containing fibers comprising, for example, a polymeric fiber containing a drug embedded, i.e., suspended, dissolved or present as a reservoir or core, therein. Such fibers when administered orally are retained in the proximal region of the gastrointestinal tract where they afford controlled release of the drug and also slowed transit thereof through the small intestine. They also serve to disperse a given drug over a greater area than, for example, a tablet or capsule and thus to minimize any irritation problems the drug might cause.

As used herein, the term "controlled release" is intended to embrace not only the concept of sustained release of drug over a prolonged period of time; i.e., relative to the time period the drug response is realized by administration of a single dose of the drug; but those of predictability and reproducibility. Thus, this invention permits both rate-wise and time-wise release of drug; i.e., drug is released at a fixed, reproducible rate over a predetermined period of time. It can, in the present instance, be said to include drug release primarily at a pre-determined locus site; namely, the proximal region of the gastrointestinal tract.

The term "drug-containing fiber" refers to any thread or threadlike structure or object, including ribbons; i.e., structures, in which one dimension, e.g. length, is significantly greater than another dimension. e.g.. width or thickness.

Said fibers can be constructed of a variety of materials such as polymers and waxes, as carriers of the drug. The construction material can be substantially inert in the use environment, or bioerodible, erodible or biodegradable in said environment.

Further the herein-described fibers can, on the basis of their construction, be referred to as matrix, laminate (or sandwich), coated matrix, reservoir or osmotic devices. Alternatively, from the standpoint of their mechanism of action they can be characterized as diffusion or osmotic devices. The matrix type includes fibers having the drug dissolved or suspended in them, and laminated devices such as those wherein the drug-containing shaped carrier is sandwiched or interposed between non-drug containing layers of a shaped carrier. The reservoir devices comprise a drug supply or drug plus excipient surrounded by a wall formed by a polymeric or wax carrier. Representative of reservoir devices are those fibers in which the drug comprises the core of what, except for the presence of the drug, is essentially a hollow fiber. both ends of which are open, or closed by appropriate sealing means (e.g., pinching, fusing, plugging). or one end of which is open and one end of which is sealed.

Representative of an osmotic device is one wherein the fiber comprises a water-insoluble, water permeable polymer, optionally comprising a porosigen dispersed therein and containing within the reservoir an osmotic enhancing agent and drug supply. Said device is optionally closed on one or both ends.

Depending upon the shape of such a fiber it can be referred to as a rod, thread, string or ribbon. It need not be straight or smooth along its greatest dimension; i.e., its length, but can be spiralled, braided or accordion-like in form to provide a greater overall surface area than that afforded by a straight elongated fiber. When viewed cross-sectionally across its shortest dimension, it can be round, square, elliptical. rectangular, or any other shape. Further, it need not be of uniform width or thickness. However, for ease of manufacture, it is advantageous to produce such fibers wherein a given dimension is substantially uniform.

In general, an axial ratio (ratio of length:thickness) of at least about 8 for the herein described fibers is favored. A minimum length of not less than 2 mm is important in order to minimize passage of the fibers through the pyloric valve. The maximum length can vary greatly depending upon the mammal to which such a fiber or fibers is to be administered. For instance such a fiber for administration to a cow could be significantly longer than one destined for use in a human. The thickness of the fiber can vary significantly so long as the above-mentioned axial ratio is satisfied. From a practical standpoint a minimum thickness of 0.1 mm is favored. Favored dimensions for human use are from 0.1-2mm in thickness and 4 mm to 40 cm in length. Preferred dimensions are 0.1 to 1 mm in thickness and 4 mm to 30 cm in length.

For large animal use, e.g., cattle, favored fiber dimensions range from 0.1 to 10 mm in thickness and from 10 mm to one meter long. For small animals, e.g., dogs, cats, the fiber dimensions are the same as those for human use. The terms "thick" and "thickness" as used herein are also intended to also mean "width" and "diameter".

The upper limit of fiber length is determined only by practical considerations related to the subject to which the fibers are to be administered and the ability of the subjects body to properly handle the particular form in which it is administered. Drug-containing fibers comprising polymers biodegradable or soluble in the use environment and those fibers comprising segments destructible in said environment permit the use of fibers of greater length than do other polymers and non-segmented fibers since their elimination poses little or no problem. The upper limit is dependent only upon the suitability of the fiber or fibers in a form suitable for easy administration to a particular subject as discussed below.

Those fibers from about 10 mm or more in length are, for ease of administration, given in the form of coils, rolls, an accordion-pleat folded strip or other form or shape which effectively reduces their overall length as to provide a shape or form orally administrable to a given subject, or inserted into capsules soluble or biodegradable in the gastrointestinal tract. The above-mentioned forms are maintained in the particular form for administration purposes by suitable constraining means. The constraining means can be any means which holds the particular form in its constrained position for the purpose of administration but which is readily removed, e.g. destroyed, dissolved, ruptured or otherwise removed in the use environment. Suitable constraining means include water soluble adhesives, gelatin string or tape, water soluble paper, enzymatically rupturable string, tape or paper.

Fibers less than about 10 mm in length can also be administered in capsules, e.g., gelatin capsules, in which one or more of the asymmetric fibers is contained. Dissolution of the capsule in the use environment releases the fiber or fibers contained therein.

In order to avoid problems in connection with elimination of fibers of about 10 mm or more in length, especially those which are not bioerodible; i.e., which do not dissolve, disintegrate or degrade in the use environment, the fibers can be constructed so as to comprise intermittent or alternating segments of a bioerodible substance or those materials enumerated above as suitable constraining means. The said substance can be selected so as to provide rapid or slow; i.e. over a period of time, destruction depending upon the residency time of the fiber in the gastrointestinal tract. It should desirably be one which undergoes destruction coincident with the desired or anticipated elimination of the fiber from the gastrointestinal tract. Said destructible substances should be so placed as to provide segments having the maximum. or less than the maximum length, to permit their passage from the gastrointestinal tract.

From a practical and utilitarian viewpoint, the use of fibers comprising bioerodible polymers: i.e., polymers which dissolve, disintegrate or degrade, is favored, especially when using fibers 10 mm or greater in length. The placement of notches or intermittent or alternating segments of a bioerodible substance along the length of such a fiber can promote its breakdown into a shorter fiber and further serve to minimize problems which might arise upon elimination of such a fiber. The notches or bioerodible segments can be evenly or unevenly placed along the length of the fiber. They should be placed so as to provide fibers the maximum length of which allows their passage from the gastrointestinal tract. Fibers having notches, intermittent or alternating segments of bioerodible substances are often favored for those 10 mm or longer in length since bending or folding of the fibers can occur. This permits use of longer fibers and favors retention.

The drug-containing fibers of this invention can be produced by methods known to those skilled in the art. In fibers wherein the drug is suspended in the fiber a suitable method comprises blending the chosen drug into an appropriate softened pre-formed fiber. A convenient way of carrying this out comprises passing a strip of said fiber through a roll mill heated to a temperature sufficient to soften the fiber but not to decompose the drug. The drug is added to the nip of the mill and the strip recirculated until satisfactory suspension of drug is achieved. The strip is then cooled and cut into elongated fibers of desired dimensions. If the strip is thicker than desired it is pressed between heated plates to provide the desired thickness. Alternatively, if, when the fiber is a polymer, the drug is stable to and non-reactive to the polymerization conditioons, it can be mixed in with the appropriate reactants prior to polymerization thereof to a polymer.

In a modification of this method, the drug and fiber, both desirably in a small mesh size, are thoroughly mixed in any conventional apparatus and the mixture then coalesced by heating to at least the softening point, and preferably above the melting point, of the fiber after which it is rolled or extruded and cut into fibers of the desired size.

A porosigen; i.e., a porosity enhancing agent can be present in the fiber to enhance the porosity thereof to the fluid of the use environment. Said porosigen can be soluble in the use environment fluid and/or biodegradable in said fluid. Its gradual removal by dissolution and/or degradation in the use environment results in formation of ducts and channels within the fiber to afford access of environmental fluid to the interior of the fiber. The porosigen must be inert to the fiber and drug and non-toxic in the use environment.

Representative porosigens are starch, lactose or other sugar, inorganic salts such as carbonates, bicarbonates, sulfates, phosphates, nitrates of the alkaline earth metals, alkali metals, ammonium salts, sodium acetate, sodium citrate, buffering agents, water soluble synthetic or semisynthetic polymers, e.g., polyethyleneglycol and hydroxypropylcellulose.

A porosigen must be used in those fibers of this invention which comprise an impermeable fiber, and can be used, if desired, in those which comprise a semi-permeable or permeable fiber to further modify the release characteristics of fibers constructed therefrom. The amount of porosigen used is dependent upon factors such as the solubility of the drug, the release rate desired and the properties of the resulting drug-porosigen containing fiber (it should remain flexible). In general, the amount of porosigen used will range from 10% to 60% by weight of the polymer.

The term "drug" as used herein includes physiologically or pharmacologically active substances which produce a systemic or localized effect or effects in a mammal, including humans and animals. Included in this term are sedatives such as phenobarbital, thiopental and sodium pentobarbital; analgesics such as codeine, morphine and mependine; local anesthetics such as procaine, lidocaine and tetracaine; levo-dopa; tranquilizers such as reserpine, chlorpromazine and fluphenazine; antibacterials such as tetracycline, oxytetracycline, penicillin, sulfonamides and chloramphenicol; antifungals such as tioconazole, griseofulvin and nystatin; antiinflammatories such as aspirin and salicylamide; nutritional agents such as essential amino acids, vitamins C and $B_{12}$; bronchodilators such as pirbuterol; diuretics such as furosemide; antihypertensives such as prazosin and doxazosin; vasodilators such as nifedipine; prostaglandins, anthelmintics, antiulcer agents; and others known to those skilled in the art.

The particular drug must, of course, be stable in the use environment; i.e., it must not be converted to a derivative which lacks the desired physiological action. Additionally, since the herein-described fibers are also useful for oral administration of more than one drug via the same fiber, or by use of a mixture of fibers, drugs to be administered must, of course, be non-reactive with each other and must not cause undesirable reactions in the subject being treated. Stability of the drug in the use environment, if a problem, can frequently be overcome by using an active derivative of the drug such as a salt, an ester or ether. Derivatization of a given drug, as by salt formation, can often be used to modify the stability and, of course, solubility characteristics of the drug.

The amount of drug used is dependent upon several factors including the fiber size, solubility in the use environment, its potency, the release rate and release period desired and the diffusion coefficient for the drug in the fiber and in the use environment. Further the amount of drug or drug and porosigen present should not be so great as to impair the flexibility of the fibers. This is readily determined by simple experiment. The desired fiber flexibility is related to the length of the fiber. Fibers should be flexible enough to prevent puncture of the stomach or intestinal wall. Long fibers (greater than about one cm) should unfold or uncoil in the stomach to assure their retention. Such fibers must be bioerodible or be flexible enough to assure their eventual passage from the stomach to the proximal small intestine. The suitability of a particular fiber length is determined by an empirical determination of gastrointestinal transit time assayed for example by x-ray radiography or scintigraphy. The flexibility of fibers found acceptable by either of these methods is then measured, if desired, by the American National Standard ANSI/ASTM Standard Test Method for Stiffness of Fabrics, D1388-64, Option B Double Cantilever Test.

The above described x-ray radiography or scintigraphy methods are also used to determine the retention time of a given fiber or fibers in the host to which they are administered.

The amount of a given drug which must be used in a fiber of this invention to achieve a given release rate, or the release rate of a given fiber of this invention, is determined by an in vitro test as those skilled in the art will recognize. In general, such a test involves placing one or more of the fibers in question in an environment approximating the ambient environment of ultimate use intended for said fiber and measuring by appropriate methodology known to those skilled in the art the amount of drug released to said environment over a given period of time and/or by determining the amount of drug remaining in the fiber after a given period of time.

As regards solubility of a given drug, there is no upper limit since the release rate can be regulated by judicious choice of polymer or polymers. As regards a lower limit of solubility, the drug should be of sufficient solubility to permit achievement of a beneficial dose of the drug from the maximum length and/or number of fibers which can be practically administered to a given subject.

The length and/or number of fibers administered to a given subject is dependent upon a number of factors, including the drug, the dose to be achieved, the time period of drug administration, the dimensions and drug content of a given fiber. The length and/or number should be sufficient to permit achievement of the dose-time response.

The dimensions, particularly the length of a given fiber, are interrelated with the amount of drug to be used. For any given amount of drug a compromise must be sought so as to maintain the length within limits which permit easy administration.

The fiber can comprise a material which is bioerodible, permeable, including microporous, semipermeable or impermeable. By permeable fiber is meant one, e.g., a polymer, which allows passage of environmental fluid and of drug. A semi-permeable fiber, on the other hand, is one which is permeable to environmental fluid and essentially impermeable to the drug or vice-versa. An impermeable fiber is one which is essentially impermeable to environmental fluid and drug. The selected fiber can, in addition, be non-erodible or bioerodible in the use environment.

The fiber which is used must be non-toxic to the mammal, including a human, to which they are to be administered, and sufficiently flexible to permit ease of administration and to avoid infliction of puncture wounds during and subsequent to administration. Representative of the polymeric materials which can be used are polyethylene, polypropylene, polystyrene, polytetrafluoroethylene, polyvinyl chloride, cellulose acetate, cellulose nitrate, cellulose triacetate, ethylene vinylacetate, polyesters, polyanhydrides, polyorthoesters, hydroxylated-ethylene vinylacetate, hydroxyethyl cellulose, acetylated hydroxyethyl cellulose, fibroin, polyglycolic acid, polylactic acid, poly(lacticglycolic) acid, cellulose acetate succinate, cellulose ethers, poly(vinylmethyl ether) copolymers, cellulose acetate laurate, polyacrylates, organosilicon polymers, methyl cellulose, poly(urethane), poly(vinylpyrrolidone), polyimides, hydrogels, polyamides and methacrylates. A polymeric system of interest comprises a polymer which is "enteric"; i.e., a polymer which is insoluble at gastric pH and soluble at intestinal pH (e.g. cellulose acetate-phthalate). The fibers can be made from extruded or woven fiber. When woven fibers are used, the thickness of the finished fiber should be from 0.1 to 2 mm as discussed above. The individual strands of the woven fiber will, of course, be $1/n^{th}$ of said thickness where n represents the number of strands.

Representative of the waxes which can be used are those which are insoluble or only poorly soluble in water, including those of plant (e.g. carnauba) or animal (e.g. beeswax) origin; paraffin (e.g. paraffin wax, microcrystalline wax), hydrogenated oils (such as soybean or castor oils). .

Mixtures of materials can also be used in order to modify the permeability characteristics. resiliency and flexibility of a given fiber as is known in the art; e.g., mixtures of ethyl cellulose/hydroxypropyl cellulose. Still further. the herein described fibers can be coated with, for example, a polymer to give a fiber comprising one or several coatings to achieve a desired release rate.

When the fiber comprises a microporous polymer it can be preformed or formed in situ by dissolution therefrom of a porosigen or drug or mixture of porosigen and drug which is suspended in finely divided form in the polymer. A microporous polymer is often favored for those fibers of this invention wherein the drug is present as the core of the fiber.

While the major factor responsible for retaining the herein described fibers in the gastrointestinal tract is their length and width the density of said fibers may also play a role. Thus, for fibers of identical length and width but of different densities, those fibers, for example, reservoir devices and hollow fibers, the density of which enables them to float in gastric juice may be retained for a longer period than are less buoyant fibers.

A procedure of value in determining the release rates of the herein described drug containing fibers is as follows. The procedure, an in vitro procedure, comprises placing the fiber or fibers in an environment approximating that of the gastrointestinal tract and measuring the amount of drug released to said environment as a function of time.

In vivo release of drug from fibers of this invention is determined by administering them to dogs and measuring the amount of drug released over a period of time by determining the amount of drug present in, for example, the animals' blood or urine.


## EXAMPLE 1

The kinetics of gastrointestinal transit of woven polyester fibers (available from Gutermann, West Germany) in rats was investigated in order to demonstrate that fibers can be retained in the upper GI tract for significant periods of time. Fasted Sprague-Dawley rats were each gavaged with 20 flexible woven polyester fibers (8mm x 0.5mm) suspended in 1 ml 0.1% Triton X-100 in water. This was followed by 1 ml water from the same syringe. The rats were then divided into groups which were subsequently unfed or were individually fed a 5 gm food pellet. The rats generally consumed the entire pellet within 15 minutes. At various times, rats were sacrificed, the stomachs were removed and opened, and the number of fibers remaining in the stomach was counted.

In Table I, the percent of fiber dose remaining in the stomach is presented for each rat, in addition to the mean and standard deviation. In fed rats, significant gastric retention of 8mm fibers was observed at 3 and 6 hours post-dose. Between 6 and 9 hours the remaining fibers were emptied from the stomach. In unfed rats, a significant but variable percentage of the fibers was retained.

This example demonstrates that small fibers are retained in the stomach for significant periods of time, and that they eventually exit from the stomach. In the unfed state, a large proportion of the fibers have exited by 3 hours, and a large animal-to-animal variability is observed, as evidenced by the large standard deviation. In this unfed state, animals have regular gastrointestinal "housekeeper" waves which move large undigested (or indigestible) material out of the stomach and down the GI tract. A group of rats will not exhibit temporally synchronized housekeeper waves; thus at the time of dosing some rats are on the verge of starting a housekeeper wave, while others may not start one for 90 minutes. Thus, in the unfed state, large intersubject variability is to be expected. It should be noted that, at 3 hours post-dose, most unfed animals have emptied only a portion of the fiber dose. Thus this dosage form exhibits a significant advantage over large ( > 2mm) non-disintegrating tablets, which will empty from the unfed stomach in an all-or-none fashion.

In the fed state, the presence of digestible material in the stomach inhibits the occurrence of housekeeper waves, and solid material must be < 2mm in diameter to pass into the small intestine. The data in Table I indicate that, in the fed state, a large proportion of the 8mm fibers is retained at 3 hours and 6 hours. The relatively small standard deviations indicate that this is a reproducible occurrence. At later times (6-9 hours), the animal is once more in the unfed state, and housekeeper waves empty the 8mm fibers in a more efficient manner.

TABLE I

Gastric retention in rats of flexible 8mm x 0.5mm woven polyester fibers. Each percentage value represents the percentage retained in the stomach of a single rat at a single time point. The mean and standard deviations are presented in parentheses.

| Time After Dosing (Hours) | % of Fibers Remaining in Stomach | |
|---|---|---|
| | Fed | UnFed |
| 3 | 100, 85, 85, 75, 75, 45 (77.5 ± 18.4) | 50, 25, 15, 10, 5, 0, 0, 0 (13.1 ± 17.3) |
| 6 | 100, 85, 70, 70, 65, 60 (75 ± 14.8) | 35, 0, 0, 0, 0, 0 (5.8 ± 14.3) |
| 9 | 50, 5, 0, 0, 0, 0 (9.2 ± 20.1) | 95, 5, 0, 0, 0, 0 (16.7 ± 38.4) |

## EXAMPLE 2

The effect of fiber length on gastric retention was investigated. Fasted rats were gavaged with 20 flexible woven polyester fibers, and gastric retention was assayed at 6 hours post-dose, as in Example 1. Particles of length 4mm, 8mm and 10mm were studied. The fiber diameter was 0.5mm in each case. In fed rats, increasing the fiber length from 4mm to 8mm resulted in increased retention (Table II). Furthermore, the variability in the data (standard deviation) decreased on going from 4mm to 8mm. An increase in length to 10mm did not result in any improvement in gastric retention, perhaps because these longer fibers can fold, thus decreasing the effective length.

In the unfed state, 4mm and 8mm fibers were not retained at 6 hours. Significant retention of 10mm fibers was observed, with large animal-to-animal variability, as explained in Example 1.

TABLE II

Gastric retention in rats of flexible woven polyester fibers at 6 hours post-dose. Mean and standard deviations are given in parentheses.

| Fiber Length (mm) | % of Fibers Remaining in Stomach | |
|---|---|---|
| | Fed | UnFed |
| 4 | 90, 70, 65, 45, 30, 10, (51.7 ± 29.1) | 30, 0, 0, 0, 0, 0 (13.3 ± 32.7) |
| 8 | 100, 85, 70, 70, 65, 60 (75 ± 14.8) | 35, 0, 0, 0, 0, 0 (5.8 ± 14.3) |
| 10 | 80, 80, 75, 65, 45, 45, 35, (63.3 ± 19.1) | 100, 70, 15, 5, 0, 0 (31.7 ± 42.7) |

EXAMPLE 3

The gastric retention of fibers composed of relatively stiff monofilament polyester elastomer fibers (Dupont Hytrel) was determined in rats. Fifty fibers were gavaged per rat, and gastric retention was assayed as in Example 1. Two fiber lengths were studied: 4mm and 10mm. In each case the fiber diameter was 0.5mm.

In the fed state, 4mm stiff Hytrel fibers exhibited significant but variable gastric retention at 3, 6 and 9 hours (Table III). The observed mean retention values were generally lower than those observed for 4mm flexible polyester fibers in Example 2. The 10mm stiff Hytrel fibers exhibited almost complete gastric retention at 6 hours post-dose. These data demonstrate that, for stiff fibers, increasing the fiber length results in a significant increase in retention. Furthermore, comparison at 6 hours post-dose of the retention of 10mm stiff Hytrel fibers (95.8 + 7.3%) with 10mm flexible woven polyester fibers (63.3 + 19.1%; Table II) indicates that stiff fibers are significantly better than highly flexible fibers, both with respect to average retention and variability.

In the unfed state, 4mm stiff Hytrel fibers exhibit almost no gastric retention (Table III). The 10mm stiff Hytrel fibers exhibited significant but highly variable retention, similar to the behavior of 10mm flexible woven polyester fibers in the unfed state (Table II). This observation is significant because it indicates that, although 10mm stiff Hytrel fibers are well retained in the fed state (95.8 + 7.3%), the fibers are capable of exiting the stomach when the stomach is empty of food.

The relative stiffness of the woven polyester thread and monofilament Hytrel fiber was quantitated using the American National Standard ANSI/ASTM Standard Test Method for Stiffness of Fabrics, D 1388-64, Option B Double Cantilever Test. The calculated flexural rigidity values were 125 mg-cm for the woven polyester thread and 1568 mg-cm for the monofilament Hytrel fiber.

TABLE III

Gastric retention in rats of stiff monofilament Hytrel polyester fibers of length 4mm and 8mm. Mean and standard deviations are given in parentheses.

| Fiber Length (mm) | Time After Dosing (Hours) | % of Fibers Remaining in Stomach | |
|---|---|---|---|
| | | Fed | UnFed |
| 4 | 3 | 80, 60, 40, 30, 10, 0 (36.7 ± 30.1) | 50, 25, 10, 0, 0, 0, 0, 0 (10.6 ± 18.2) |
| 4 | 6 | 32, 30, 30, 20, 10, 5 (21.2 ± 11.5) | 0, 0, 0, 0, 0, 0 (0) |
| 4 | 9 | 75, 60, 10, 5, 0, 0 (25 ± 33.5) | Not done |
| 10 | 6 | 100, 100, 98, 98, 98, 98, 96, 78 (95.8 ± 7.3) | 94, 80, 60, 0, 0, 0 (33.4 ± 42.8) |

## EXAMPLE 4

The relative retention of 4mm fibers prepared from 3 materials was investigated. These materials were monofilament ethylenevinylacetate (EVA) (Dupont Elvax-150, 33% vinylacetate content), monofilament Hytrel, and woven polyester (WPE). The monofilament EVA threads were similar in flexibility to the woven polyester fibers, and more flexible than the monofilament polyester fibers. Using the ANSI/ASTM Stiffness Test D-1388-64-B, flexural rigidity values of 125 mg-cm, 110 mg-cm, and 1568 mg-cm were determined for the woven polyester, monofilament EVA, and monofilament Hytrel fibers, respectively. EVA differed from the other two materials in that EVA floated in aqueous suspensions, while the other two materials sank.

Fasted rats were gavaged with fibers, and gastric retention was assayed as in Example 1. In the fed state, gastric retention of EVA fibers was superior to that of stiff Hytrel or flexible woven polyester at 3, 6 and 9 hours (Table IV). In the unfed state, significant retention was observed at 3 hours, with marginal retention at 6 hours. In the unfed state, variability was high, as also observed in the preceding examples and explained in Example 1.

The superior retention of EVA (at 4mm) demonstrates that properties other than flexibility and length can affect the kinetics of retention of fibers. In this example, the low density of the EVA fibers is probably involved in their superior retention.

## TABLE IV

Gastric retention in rats of 4mm x 0.5mm fibers composed of monofilament ethylenevinylacetate (EVA), monofilament Hytrel polyester or woven polyester (WPE). Values in parenthesis represent mean and standard deviations.

A. Fed State

### % of Fibers Remaining in Stomach at X hour Post-dose

| Material | 3 hours | 6 hours | 9 hours |
|---|---|---|---|
| EVA | 100, 100, 95, 90, 85, 20 (81.7 ± 30.8) | 100, 95, 80, 70, 65, 55 (77.5 ± 17.5) | 70, 60, 45, 35, 30, 20 (43.3 ± 18.9) |
| Hytrel | 30, 60, 40 30, 10, 0 (36.7 ± 30.1) | 32, 30, 30 20, 10, 5 (21.2 ± 11.5) | 75, 60, 10, 5, 0, 0 (25 ± 33.5) |
| WPE | 55, 45, 30, 25, 25, 0 (30 ± 19.0) | 90, 70, 65, 45, 30, 10 (51.7 ± 29.1) | 90, 60, 50, 30, 15, 0 (40.8 ± 32.6) |

B. Unfed State

| Material | 3 hours | 6 hours | 9 hours |
|---|---|---|---|
| EVA | 60, 25, 20, 10, 5, 0, 0 (17.1 ± 21.2) | 55, 0, 0, 0, 0, 0 (9.2 ± 22.5) | not done |
| Hytrel | 50, 25, 10, 0 0, 0, 0, 0 (10.6 ± 13.2) | 0, 0, 0, 0, 0, 0 (0) | not done |
| WPE | 0, 0, 0, 0, 0, 0 (0) | 80, 0, 0, 0, 0, 0 (13.3 ± 32.7) | 0, 0, 0, 0, 0, 0 (0) |

EXAMPLE 5

Gastric retention of long flexible fibers in unfed dogs was assessed using x-ray radiography. A 25 cm piece of hollow Silastic (Dow Corning Corp., Midland, Michigan) tubing (diameter 1 mm) was filled with a saturated solution of potassium iodide, which served as a radiopaque agent. The ends were tied, and the fiber was coiled and placed in a #00 gelatin capsule. A beagle dog was orally dosed with the capsule, and the GI transit of the fiber was followed by x-ray radiography at 10 minutes, 30 minutes, 1 hour, 1.5 hours, 2.5 hours, 3.5 hours, 5 hours, 6 hours and 8 hours. The fibers was clearly retained in the stomach for 6 hours. At 8 hours, the fiber was visualized in the small intestine. This gastric retention is particularly significant because it was observed in the unfed state, which is characterized by housekeeper waves which remove indigestible material from the stomach. Control experiments were carried out in which the GI transit of radiopaque non-disintegrating standard round tablets was assessed in beagle dogs. Two tablet sizes were dosed: 6.4mm diam x 2.5mm thickness, and 4.0mm diam x 1.5mm thickness. Tablets of both sizes were consistently observed to exit from the stomach in less than approximately 2.5 hours.

Gastric retention of a long flexible fiber was also assessed in a fed dog. A 25cm piece of radiopaque Silastic tubing (1 mm diam) was prepared and dosed as described above. Immediately after dosing, the dog was fed her normal daily ration of dry food and was allowed access to water. X-ray radiography indicated that the fiber was present in the stomach at 0.33 hour, 5.33 hours and 18 hours post-dose. At 20.33 hours post-dose, the fiber was observed to be in the process of exiting from the stomach through the pylorus.

This example demonstrates that long flexible fibers can be retained in the stomach for extended time periods in both the fed and unfed states, and that such fibers eventually exit from the stomach.

EXAMPLE 6

Gastric retention of a fiber in a beagle dog was assessed under the condition in which the fiber was dosed to an unfed dog which was fed at a later time. A beagle dog was dosed with a 30 cm x 1 mm radioopaque fiber as in Example 5. X-ray radiography at 15 minutes, 2.5 hours, 5 hours and 7.5 hours indicated that the fiber was retained in the stomach. At 8 hours post-dose, the dog was fed her normal daily food ration. X-ray radiography at 24 hours post-dose indicated that the fiber was still retained in the stomach.

EXAMPLE 7

The effect of fiber length on gastric retention of fibers in unfed dogs was assessed using X-ray radiography. Gastric retention of a radiopaque non-disintegrating tablet was concurrently monitored as an internal control. Radiopaque Silastic tubing was prepared as in Example 5. Beagle dogs were dosed with a #00 gelatin capsule which contained (a) a 4.0 mm x 1.5 mm radiopaque non-disintegrating standard round convex tablet, and (b) a silastic fiber with outer diameter 1 mm and length 10 cm, 20 cm or 30 cm. Gastric retention data is presented in Tables V, VI and VII for dogs A, B and C, respectively. All three dogs almost always retained the fiber longer than the tablet. Comparison of the three fiber lengths indicates no significant effect of length on gastric retention, over the fiber length range 10-30 cm. Thus, the minimum required fiber length for retention is under 10 cm, for Silastic fibers of the type used. These fibers were very flexible, and the observations reported in Tables V-VII indicate the effect of fiber length only for very flexible fibers.

The average gastric retention time for both tablets and fibers differed among the three dogs. For example, dog C consistently retained both tablets and fibers for longer time periods than dogs A and B. However, for all dogs, fibers were consistently retained longer than tablets. In all cases, fibers eventually exited the stomach.

12

Table V.  Gastric Retention of Silastic Fibers of Various Lengths (1 mm diameter) and Non-Disintegrating Tablets in Beagle Dog A.

| Fiber Length (cm) | Gastric Retention[a] at Various Times Post-Dose | | | | | | |
|---|---|---|---|---|---|---|---|
| | 10 min | 1 hr | 2 hr | 3 hr | 4 hr | 5 hr | 6 hr |
| 10 | I/I | O/I | O/O | - | - | - | - |
| 10 | I/I | I/I | I/I | O/I | O/I | O/I | O/I |
| 10 | I/I | I/I | O/I | O/O | - | - | - |
| 20 | I/I | O/O | - | - | - | - | - |
| 20 | I/I | I/I[b] | O/I | O/O | - | - | - |
| 20 | I/I | O/I[b] | O/O | - | - | - | - |
| 30 | I/I | O/I | O/I | O/I | O/O | - | - |
| 30 | I/I | I/I | I/I | I/I | O/I | O/I | O/I |
| 30 | I/I[b] | O/I[b] | O/O | - | - | - | - |

[a]  I/I = tablet in/fiber in; O/I = tablet out/fiber in; O/O = tablet out/fiber out

[b]  fiber in both stomach and upper small intestine

Table VI. Gastric Retention of Silastic Fibers of Various Lengths (1 mm diameter) and Non-Disintegrating Tablets in Beagle Dog B.

| Fiber Length (cm) | Gastric Retention[a] at Various Times Post-Dose | | | | | | |
| :---: | :---: | :---: | :---: | :---: | :---: | :---: | :---: |
| | 10 min | 1 hr | 2 hr | 3 hr | 4 hr | 5 hr | 6 hr |
| 10 | I/I | O/O | - | - | - | - | - |
| 10 | I/I | O/I[b] | O/I[b] | O/O | - | - | - |
| 10 | I/I | O/I[b] | O/O | - | - | - | - |
| 20 | I/I | I/I | O/O | O/O | O/O | O/O | O/O |
| 20 | I/I | O/I[b] | O/I[b] | O/O | - | - | - |
| 20 | I/I | I/I | O/I[b] | O/I[b] | O/I[b] | O/O | - |
| 30 | I/I | I/I[b] | O/I[b] | O/I[b] | O/I | O/I | O/O |
| 30 | O/I | O/I[b] | O/I[b] | O/O | O/O | - | - |
| 30 | I/I | I/I | O/O | O/O | O/O | O/O | - |

[a] I/I = tablet in/fiber in; O/I = tablet out/fiber in; O/O = tablet out/fiber out

[b] fiber in both stomach and upper small intestine

14

Table VII. Gastric Retention of Silastic Fibers of Various Lengths (1 mm diameter) and Non-Disintegrating Tablets in Beagle Dog C.

| Fiber Length (cm) | Gastric Retention[a] at Various Times Post-Dose | | | | | | |
|---|---|---|---|---|---|---|---|
| | 10 min | 1 hr | 2 hr | 3 hr | 4 hr | 5 hr | 6 hr |
| 10 | I/I | I/I | I/I | I/I[b] | I/I[b] | I/I[b] | O/I[b] |
| 10 | I/I | I/I | I/I | I/I | O/I | O/I | O/I |
| 10 | I/I | I/I | I/I | I/I | I/I | O/I[b] | O/I[b] |
| 20 | I/I | I/I | I/I | I/I | I/I[b] | O/I[b] | O/I[b] |
| 20 | I/I | I/I | O/I[b] | O/I[b] | O/I[b] | O/I[b] | O/O |
| 20 | I/I | I/I | I/I | O/I[b] | O/I[b] | O/I[b] | O/I[b] |
| 30 | I/I | I/I | O/I | O/I | O/I[b] | O/I[b] | O/I[b] |
| 30 | I/I | I/I | I/I | I/I | I/I | I/I | I/I |
| 30 | I/I | I/I | O/I | O/I | O/I | O/I | O/I[b] |

[a] I/I = tablet in/fiber in; O/I = tablet out/fiber in; O/O = tablet out/fiber out

[b] fiber in both stomach and upper small intestine

## EXAMPLE 8

The effect of fiber stiffness on gastric retention was assessed by dosing unfed beagle dogs with polyethylene fibers, which were stiffer than the Silastic fibers described in Examples 5, 6 and 7. The relative stiffness of the polyethylene (1 mm outer diam.) and Silastic (1 mm outer diam.) fibers was quantitated using the American National Standard ANSI/ASTM Standard Test Method for Stiffness of Fabrics, D 1388-64, Option B Double Cantilever Test. The calculated flexural rigidity values were 1182 mg-cm for the Silastic fiber and 27,305 mg-cm for the polyethylene fiber.

Beagle dogs were dosed with a #00 gelatin capsule containing (a) a 4.0 mm x 1.5 mm radiopaque non-disintegrating standard round convex tablet, and (b) a coiled radiopaque polyethylene fiber (10 cm x 1 mm).

Gastric retention data is presented in Table VIII. In all three dogs studied, a 10 cm polyethylene fiber was retained in the stomach for at least 1 day. In two cases (dogs A and B), the fiber was retained in the animal for at least 6 days. In all cases, the fiber eventually exited from the animal. Comparison of these results with those obtained with 10 cm Silastic threads (Tables V, VI, VII) demonstrates that increasing the stiffness of the fiber results in increased gastric retention.

Table VIII. Gastric Retention of Polyethylene Fibers (10 cm x 1 mm) and Non-disintegrating Tablets in Beagle Dogs.

| Time Post-Dose | Gastric Retention[a] | | |
|---|---|---|---|
| | Dog A | Dog B | Dog C |
| 10 min | I/I | I/I | I/I |
| 1 hr | I/I | O/I | I/I |
| 2 hr | O/I | O/I | O/I |
| 3 hr | O/I. | O/I | O/I |
| 4 hr | O/I | O/I | O/I |
| 5 hr | O/I | O/I | O/I |
| 6 hr | O/I | O/I | O/I |
| 1 day | O/I | O/I | O/I |
| 2 days | NM[c] | NM | O/O |
| 6 days | O/I | O/O[b] | NM |
| 13 days | O/O | O/O | NM |

[a] I/I = tablet in/fiber in; O/I = tablet out/fiber in; O/O = tablet out/fiber out

[b] fiber visible in small intestine.

[c] not measured

EXAMPLE 9

The ability of a gastrically-retained fiber dosage form to alter the pharmacokinetics of a drug was assessed. A drug-releasing fiber was prepared as follows. A 31 cm x 1 mm hollow Silastic fiber was sealed at one end. The first 15 cm (sealed end) contained a suspension of NaCl (350 mg/ml) in a saturated aqueous solution of NaCl. The terminal 14 cm (open end) contained a solution of doxazosin mesylate (an antihypertensive drug) in aqueous 60% (w/w) citric acid. The two aqueous compartments were separated by a 1 cm segment of Mazol vegetable oil. The drug-loaded fiber was coiled around a #3 gelatin capsule, and placed in a #00 gelatin capsule. Fibers of this type released doxazosin mesylate into simulated gastric fluid at a constant rate.

Fasted beagle dogs were orally dosed with 1 mg doxazosin mesylate in four types of formulations: (1) the fiber dosage form described above, (2) small (1 mm) controlled release beads, (3) a slowly eroding tablet, and (4) a quickly releasing tablet. The dogs were fed their normal daily food ration at 2 hours post-dose. Blood was collected at various times after dosing, and was assayed for the presence of doxazosin. using a high performance liquid chromatography assay. The results are summarized in Table IX.

Table IX. Pharmacokinetics of doxazosin in beagle dogs, after dosing with a standard quickly releasing tablet, a controlled release eroding tablet, three different controlled release bead preparations, and a gastrically-retained fiber dosage form of the present invention.

| Formulation | Avg. $T_{max}$ (hr) | Avg. $C_{max}$ (ng/ml) | % Bio-availability | n |
|---|---|---|---|---|
| Quick release tablet | 1.1 | 16 | 100 | 4 |
| CR-beads-A | 2.7 | 9.6 | 83 | 4 |
| CR-beads-B | 4.3 | 6.0 | 51 | 4 |
| CR-beads-C | 3.5 | 4.1 | 42 | 4 |
| CR-eroding tablet | 3.5 | 7.3 | 66 | 4 |
| fiber | 8.0 | 7.6 | 84 | 2 |

CR = controlled release

After dosing with a standard quickly releasing tablet, a maximum doxazosin blood level ($C_{max}$) was observed at 1.1 hour post-dose ($T_{max}$). Three controlled release bead preparations were dosed, which differed in their drug-release rates. Bead formulation A released drug more quickly in vitro than bead formulation B, which released drug more quickly than bead formulation C (data not shown). After dosing with bead formations A, B and C, maximum doxazosin blood levels were observed at 2.7, 4.3 and 3.5 hours, respectively. Bead formulation A had released most or all of its drug by 2.7 hours ($T_{max}$), reflected in the high bioavailability (83%) relative to the quickly releasing tablet. Bead formulations B and C exhibited lower bioavailabilities (51%, 42%), indicating that little or no drug was absorbed at times after $T_{max}$ (4.3 hours, 3.5 hours). These results indicate that the dog exhibits a "colonic window of absorption" for doxazosin, because the transit time of 1 mm beads through the stomach and small intestine is known to be approximately 3-4 hours (Davis, S.S., Hardy, J.G. and Fara, J.W. (1986) Gut 27, 886-892). Thus bead formulations B and C release drug which is absorbed efficiently, until they pass into the colon at approximately 3-4 hours post-dose. At that time, doxazosin blood levels start to fall because absorption of this drug from the colon is inefficient, for reasons which have not been determined. The controlled release eroding tablet formulation behaves similarly, with $T$max 3.5 hours and a bioavailability of 66% relative to the quickly .releasing tablet. This observation is also consistent with the presence of a colonic window of absorption for doxazosin in the dog. The mouth to colon transit time of a tablet of this type in fasted dogs is expected to be 3-4 hours (Davis et al., loc. cit.).

Dogs dosed with doxazosin mesylate in fiber dosage forms exhibited significantly different doxazosin pharmacokinetics. As shown in Table IX, a $T$max at 8.0 hours was observed, with a bioavailability of 84%. The observations reported in Example 6 indicate that the doxazosin-containing Silastic fiber would be expected to remain in the stomach for a significant time period (as long as 24 hours), under the feeding conditions of this experiment. The use of this dosage form has clearly eliminated the 3-4 hours "window of absorption". The high bioavailability (84%) suggests that the fiber slowly released all of its drug in the uper GI tract, where doxazosin absorption is efficient. At approximately 8 hours post-dose, the fiber was totally depleted of drug.

This Example demonstrates conclusively that a gastrically-retained fiber dosage form can be used to eliminate a "window of absorption", and to improve bioavailability. This Example does not limit in any way the design of the controlled release aspects of the fiber dosage form, the materials from which the dosage form can be constructed, or the final presentation of the dosage form.

## Claims

1. Drug-containing fibers for retention in the gastrointestinal tract as use environment comprising a drug-containing polymeric fiber wherein the axial ratio of the fiber is at least about 8 and wherein the minimum dimension is at least about 0.1mm and said fiber if not less than 2mm in length.

2. Drug-containing fiber according to claim 1 wherein the polymer is permeable to passage of the use environment fluid, said polymer optionally containing a porosigen.

3. Drug-containing fiber according to claim 1 wherein the drug is present as a core, a solution or suspension in said fiber.

4. Drug-containing fiber according to claim 1 wherein the polymer is bioerodible in the fluid present in the use environment.

5. Drug-containing fiber according to claim 1 wherein the polymer is impermeable to passage of drug and the use environment fluid and wherein a porosigen is present in the polymer.

6. Drug-containing fiber according to claim 5 wherein the polymer is bioerodible in the use environment fluid.

7. Drug-containing fiber according to claim 1 wherein the polymer is a microporous polymer.

8. Drug-containing fiber according to claim 7 wherein the drug is present as a core, a solution or suspension in said fiber.

9. Use of drug containing fibers of claim 1 for controlled release of a drug in the gastrointestinal tract which comprises oral administration to said gastrointestinal tract of an effective amount of said drug-containing fiber.

10. Process for production of a drug-containing polymeric fiber for retention in the gastrointestinal tract which comprises

(i) introducing said drug into said fiber; or

(ii) blending said drug, optionally with a porosigen, into said fiber, said fiber being in a softened condition, followed by cooling the resulting drug/fiber; or

(iii) blending said drug, optionally with a porosigen, into the polymer, said polymer being in a softened condition, and then cooling and converting the drug/polymer blend to the desired dimensions; or

(iv) mixing the drug, optionally with a porosigen, with the appropriate reactants required to produce said polymer prior to polymerization of said reactants, followed by polymerization thereof and conversion of the drug/polymer thus produced to fibers of the desired dimensions.

11. A process according to claim 10 wherein the polymer is permeable to passage of the use environment fluid.

12. A process according to claim 10 wherein the polymer is a bioerodible polymer.

13. A process according to claim 10 wherein the polymer is impermeable to passage of drug and the use environment fluid.

14. A process according to claim 10 wherein the polymer is a microporous polymer.

15. A process according to claim 10, step (iii), wherein the polymer is impermeable to the environmental fluid and a porosigen is present.